# EUROPEAN PATENT APPLICATION

(11) **EP 1 939 158 A1**
(43) Date of publication of application: **02.07.2008**
(21) Application number: 06256432.3
(22) Date of filing: 19.12.2006
(51) Int. Cl.: C07C 6/10, C07C 9/08, C07C 9/10, C07C 9/14

(54) **Process for converting methane into higher alkanes**

(71) Applicant: BP OIL INTERNATIONAL LIMITED, Sunbury-on-Thames, Middlesex TW16 7BP (GB)
(72) Inventor: The designation of the inventor has not yet been filed
(74) Representative: De Kezel, Eric

(57) **Abstract**

The present invention relates to a process for converting methane into higher alkanes having at least 3 carbon atoms (C₃₊), preferably at least 4 carbon atoms (C₄+). The process comprises: a stage (1) for non-oxidative methane coupling, comprising contacting methane with a metal catalyst (C1) capable of producing, in contact with alkane, reactions involving the splitting and recombining of C-C and/or C-H and/or C-metal bonds, so as to form a mixture (M1) comprising ethane and hydrogen, optionally a stage (2) for separating ethane from the mixture (M1) so as to isolate the ethane, a stage (3) for ethane metathesis, comprising contacting the ethane thus isolated or optionally the mixture (M1) with a metal catalyst (C2) capable of producing, in contact with alkane, reactions involving the splitting and recombining of C-C and/or C-H and/or C-metal bonds, said catalyst being identical to or different from catalyst (C1), so as to form a mixture (M2) comprising methane and higher alkanes (C₃₊), preferably (C₄₊), and a stage (4) for separating and isolating said higher alkanes from the mixture (M2). Methane separated and isolated in stage (4) is preferably recycled into stage (1). Hydrogen produced in stage (1) is preferably separated and isolated in stage (2) and then used for various applications and stages, e.g. for producing thermal and/or electrical energies preferably employed to run the process.

## Description

The present invention relates to a process for converting methane into higher alkanes having in particular at least 3 carbon atoms (C₃₊), preferably at least 4 carbons atoms (C₄₊).

Methane is one of the hydrocarbons most widely distributed in nature in the world, and the methane deposit sites can be very far removed from one another and from the consumption areas. It follows that the methane often has to be transported over long distances. Methane is however difficult to transport by virtue of its gaseous nature. By converting it from the gaseous state into the liquid state, the methane can then be transported more easily, but this represents very high capital and energy costs.

A solution would be to convert the methane at low cost into higher alkanes which would be liquid under standard temperature and pressure conditions or conditions relatively close to the latter. This is the solution which the present invention proposes.

Processes for converting methane into liquid fuels by an oxidising route have already been known for a long time. The processes comprise first of all a reforming stage for converting the methane into a mixture of carbon monoxide and hydrogen, also called "synthesis gas" or "syngas", and then one or more stages for converting said mixture into gasoline and other easily transportable fuels. However, said processes require a great deal of energy and high investment in particular in order to achieve very high temperatures that can exceed 800 °C. These processes, in addition to being very expensive to build and to operate, also generate substantial quantities of green house causing carbon dioxide. Furthermore, the "carbon atom efficiency" of such processes is undesirably low.

In addition, International Patent Application WO 98/02244 discloses a process for converting alkanes into their higher and lower homologues. According to the process, one or more starting alkanes are reacted on a metal catalyst suitable for alkane metathesis and chosen in particular from metal hydrides and organometallic compounds fixed to a solid support. However, the process does not relate to conversion of methane, but to conversion of alkanes having at least 2 carbon atoms. Various conversions are shown as examples, in particular those of ethane, propane, butane or isobutane respectively into their higher and lower homologues.

International Patent Applications WO 01/04077 and WO 03/066552 disclose processes for preparing alkanes by reacting methane with at least one other starting alkane in the presence of an alkane metathesis metal catalyst suitable for producing, in contact with alkane, reactions involving the splitting and recombining of carbon-carbon and/or carbon-hydrogen and/or carbon-metal bonds, especially a metal catalyst chosen from the catalysts mentioned in International Patent Application WO 98/02244. Said reaction, known under the term "methane-olysis" permits the preparation of at least one or two alkanes having a number of carbon atoms less than or equal to that of the starting alkane and at least equal to 2. Various methane-olysis reactions are shown as examples, in particular by reactions of the methane with ethane, propane or n-butane. It appears that methane-olysis of ethane leads to formation of methane and ethane, that is to say to formation of the starting products of the reaction. In addition, it is shown that methane-olysis of propane leads to formation of ethane, and that of n-butane permits ethane and propane to be prepared. However, the methane-olysis process involves the utilisation of methane necessarily with another starting alkane, and leads to producing alkanes whose number of carbon atoms is not higher than that of starting alkane.

International Patent Applications WO 03/104171 discloses a process for converting methane into ethane by contacting methane with an alkane metathesis metal catalyst chosen in particular from the catalysts mentioned in International Patent Application WO 98/02244. The process employs a methane coupling reaction, also called a methane homologation reaction. It is stated that the process forms ethane with a very high selectivity by weight compared with the carbon-containing products formed, for example a selectivity by weight that can reach 99% or even higher. In addition, it is suggested that the ethane thus prepared can then be used in other processes for upgrading of the ethane, such as dehydrogenation, catalytic cracking or thermal cracking, in order to prepare more particularly olefins, such as ethylene. International Patent Application WO 03/104171 does not envisage a process for the preparation of higher alkanes beyond ethane, such as higher alkanes (C₃₊), preferably (C₄₊).

International Patent Application WO 2006/060692 discloses supported metal clusters comprising one or more metals of Groups 4, 5 and/or 6 of the Periodic Table of the Elements, and which can be used as catalysts for alkane reactions. More particularly, the supported metal clusters are capable of being used to catalyze the metathesis (also referred to as disproportionation) of alkanes, conversions of alkanes with each other to give alkane products with molecular weights different from those of the starting alkanes, and conversion of methane to higher molecular-weight alkanes. The only alkane reactions described and shown as examples relate to a self-metathesis of ethane to give propane and methane, and a conversion of methane and n-butane (i.e. methane-olysis of n-butane) to give propane and ethane. However, the Application neither discloses, nor suggests a process for converting methane into higher alkanes (C₃₊), preferably (C₄₊). Indeed, it is shown that methane reacting with other starting alkane (e.g. n-butane) leads to formation of final alkanes (e.g. propane and ethane) with molecular weight lower than that of the starting alkane.

Thus, it appears that none of the above-mentioned patent applications proposes a process for converting methane into higher alkanes having at least 3 carbon atoms (C₃+), preferably at least 4 carbon atoms (C₄₊), and capable more particularly of being easily transportable in liquid form, e.g. under standard temperature and pressure conditions or conditions close to the latter.

A process has now been found for converting methane into higher alkanes (C₃₊), preferably (C₄₊) and capable in particular of being easily transportable in liquid form, e.g. under standard temperature and pressure conditions or conditions close to the latter. The process has the advantage of being able to produce from methane, higher alkanes (C₃₊), preferably (C₄₊). It also has the advantage of being able to produce said higher alkanes with a high degree of purity, in particular free of alkenes, alkynes, aromatic compounds, carbon monoxide, carbon dioxide, sulfur- or nitrogen-containing products. It also has the advantage of being able to produce said higher alkanes with a narrow distribution of the number of carbon atoms, in particular ranging for example from 3 to 15 or preferably from 4 to 15, particularly from 3 to 13 or preferably from 4 to 13, more particularly from 3 to 10 or preferably from 4 to 10, or even from 3 to 8 or preferably from 4 to 8 carbon atoms. The present invention has, in addition, the advantage of proposing a process by a non-oxidising route, of being simple and direct, and consequently of being relatively inexpensive. In addition, one of the most important advantages of the process of the present invention relates to the potential for an extremely high "carbon atom efficiency" which can approach 95% or higher for converting methane into easily transportable mixture of higher alkanes.

More particularly, the present invention relates to a process for converting methane into higher alkanes having at least 3 carbon atoms (C₃₊), preferably at least 4 carbon atoms (C₄₊), characterised in that it comprises the following stages:
- a stage (1) for non-oxidative methane coupling, comprising contacting methane with a metal catalyst (C1) capable of producing, in contact with alkane, reactions involving the splitting and recombining of carbon-carbon and/or carbon/hydrogen and/or carbon metal bonds, so as to form a mixture (M1) comprising ethane and hydrogen,
- optionally a stage (2) comprising separating ethane from the mixture (M1), so as to isolate the ethane,
- a stage (3) for ethane metathesis, comprising contacting the ethane isolated in stage (2) or the mixture (M1) obtained in stage (1) with a metal catalyst (C2) capable of producing, in contact with alkane, reactions involving the splitting and recombining of carbon-carbon and/or carbon-hydrogen and/or carbon-metal bonds, said catalyst being identical to or different from the catalyst (C1), so as to form a mixture (M2) comprising methane and higher alkanes (C₃₊), preferably (C₄₊), and
- a stage (4) comprising separating the higher alkanes (C₃₊), preferably (C₄₊) from the mixture (M2), so as to isolate said higher alkanes.

Stage (1) of the process comprises contacting methane with a metal catalyst (C1) capable of producing, in contact with alkane, reactions involving the splitting and recombining of carbon-carbon and/or carbon-hydrogen and/or carbon-metal bonds.

The catalyst (C1) can be chosen from metal catalysts which are capable of alkane metathesis. It can be chosen particularly from supported metal clusters, and preferably from metal hydrides, organometallic compounds and organometallic hydrides, in particular supported on, and more specifically grafted onto, a solid support.

The catalyst (C1) can be chosen in particular from supported metal clusters comprising one or more metals of Groups 4, 5 and/or 6 of the Periodic Table of the Elements, preferably tantalum. The Periodic Table (here and hereafter) is that proposed by IUPAC in 1991 and, for example, published by CRC Press, Inc. (USA) in « CRC Handbook of Chemistry and Physics », 76th Edition (1995-1996), by David R. Lide. The supported metal clusters generally comprise a solid support and a metal cluster, and preferably comprise bonding between the metals of the metal cluster, and also bonding between the metal cluster and the solid support. Thus, the metal atom of the metal cluster can be bonded to at least one other metal atom of the metal cluster, preferably to six or fewer metal atoms of the metal cluster. The metal atom of the metal cluster can be bonded in addition to at least one hydrocarbon radical and/or to at least one hydrogen atom. The solid support can be chosen from a metal oxide, a refractory oxide, a molecular sieve, a zeolite, a metal phosphate and a material comprising a metal and oxygen, preferably silica. More particularly, the supported metal clusters can comprise tantalum (as a metal cluster) and silica (as a solid support), and preferably comprise bonding between tantalum and tantalum, and also bonding between tantalum and silica, in particular bonding between tantalum and oxygen on the silica surface. The catalyst (C1) can be chosen from the supported metal clusters described in International Patent Application WO 2006/060692, and prepared according to any methods described in said Application.

The catalyst (C1) is preferably chosen from metal hydrides, organometallic compounds and organometallic hydrides, particularly supported on, and more specifically grafted onto, a solid support. It can comprise at least one metal chosen from lanthanides, actinides and metals of Groups 2 to 12, preferably transition metals of Groups 3 to 12, more particularly Groups 3 to 10 of the Periodic Table of the Elements. In particular, the metal of the catalyst can be chosen from scandium, yttrium, lanthanum, titanium, zirconium, hafnium, vanadium, niobium, tantalum, chromium, molybdenum, tungsten, rhenium, ruthenium, osmium, nickel, iridium, palladium, platinum, cerium and neodymium. It can be chosen, preferably, from yttrium, titanium, zirconium, hafnium, vanadium, niobium, tantalum, chromium, molybdenum, tungsten, rhenium, ruthenium and platinum, and more especially from niobium, tantalum, molybdenum, tungsten and rhenium.

The catalyst (C1) chosen from metal hydrides and preferably from organometallic compounds and organometallic hydrides, can comprise in particular at least one hydrocarbon radical (R), either saturated or unsaturated, linear or branched, having preferably from 1 to 20, in particular from 1 to 14 carbon atoms. The hydrocarbon radicals (R) can be chosen from alkyl radicals, in particular either saturated or unsaturated, linear or branched, aliphatic or alicyclic, for examples alkyl, alkylidene or alkylidyne radicals, particularly from C₁ to C₁₀, from aryl radicals, in particular from C₆ to C₁₂, and from aralkyl, aralkylidene or aralkylidyne radicals, particularly from C₇ to C₁₄. Thus, the metal atom of the catalyst (C1) can preferably be bonded to at least one hydrogen atom and/or to at least one hydrocarbon radical (R), in particular by a single, double or triple carbon-metal bond.

The catalyst (C1) preferably selected from metal hydrides, organometallic compounds and organometallic hydrides, can furthermore comprise one or more ligands, such as "ancillary" ligands, preferably comprising at least one oxygen atom and/or at least one nitrogen atom, and particularly chosen from oxo, alkoxo, aryloxo, aralkyloxo, nitrido, imido and amido ligands. Generally, by oxo, alkoxo, aryloxo, aralkyloxo, nitrido, imido and amido ligands are respectively meant:
- a divalent oxo radical of general formula : = O
- a monovalent alkoxo, aryloxo or aralkyloxo radical of general formula : - OR'
- a trivalent nitrido radical of general formula: ≡ N
- a divalent imido radical of general formula: = NR" , and
- a monovalent amido radical of general formula : - NR¹R² ,
general formulae in which O represents an oxygen atom, N represents a nitrogen atom, R' represents an hydrogen atom or a monovalent hydrocarbon radical selected respectively from alkyl, aryl and aralkyl radicals, R" represents an hydrogen atom or a monovalent hydrocarbon radical, and R' and R², being identical or different, represent an hydrogen atom or a monovalent hydrocarbon radical. More particularly, R', R", R¹ and R² can be a monovalent hydrocarbon radical, either saturated or unsaturated, linear or branched, comprising from 1 to 20, preferably from 1 to 14 carbon atoms, and particularly chosen from alkyl radicals, preferably from C₁ to C₁₀, aralkyl radicals, preferably from C₇ to C₁₄, and aryl radicals, preferably from C₆ to C₁₂.

Thus, when the catalyst (C1) selected in particular from metal hydrides, organometallic compounds and organometallic hydrides comprises at least one of the above-mentioned ligands, the metal of the catalyst is not only bonded to at least one hydrogen atom and/or to at least one hydrocarbon radical (R), but also to at least one of these ligands, e.g. to the O atom of the oxo radical by a double bond, or to the O atom of the alkoxo, aryloxo or aralkyloxo radical (OR') by a single bond, or to the N atom of the nitrido radical by a triple bond, or to the N atom of the imido radical (NR") by a double bond, or to the N atom of the amido radical (NR¹R²) by a simple bond. Generally the presence of the oxo, alkoxo, aryloxo, aralkyloxo, nitrido, imido and/or amido ligands in the catalyst (C1) favourably influences the behaviour of the catalyst in the methane coupling reaction of stage (1).

In addition, the catalyst (C1) chosen from metal hydrides, organometallic compounds and organometallic hydrides is preferably supported on, or more particularly grafted onto, a solid support that can be chosen from metal or refractory oxides, sulfides, carbides, nitrides and salts, and from carbon, mesoporous materials, organic/inorganic hybrid materials, metals and molecular sieves. The solid support is in particular chosen from silicas, aluminas, silica-aluminas and aluminium silicates, either simple or modified by other metals, and from zeolites, clays, titanium oxide, cerium oxide, magnesium oxide, niobium oxide, tantalum oxide and zirconium oxide. It can be chosen, preferably, from silicas, aluminas, silica-aluminas and aluminium silicates, either simple or modified by other metals. The solid support can have a specific surface area (B.E.T.) measured according to the standard ISO 9277 (1995) which is chosen in a range of from 0.1 to 5000 m²/g, preferably from 1 to 3000 m²/G, more particularly from 1 to 1000 m²/g. It is preferred to use a catalyst (C1) grafted onto a solid support and in which the metal atom can be bonded to at least one atom of hydrogen, to at least one hydrocarbon radical (R), and also to at least one of the atoms of the support, in particular to at least one atom of oxygen, sulfur, carbon or nitrogen of the support, in particular when the support is chosen from metal or refractory oxides, sulfides, carbides, nitrides and salts. The catalyst (C1) can be preferably a metal hydride or an organometallic hydride grafted onto a solid support, more particularly chosen from niobium hydrides or organometallic niobium hydrides grafted onto silica, tantalum hydrides or organometallic tantalum hydrides grafted onto silica, molybdenum hydrides or organometallic molybdenum hydrides grafted onto silica, tungsten hydrides or organometallic tungsten hydrides grafted onto alumina, and rhenium hydrides or organometallic rhenium hydrides grafted onto silica.

The catalyst (C1) selected from metal hydrides, organometallic compounds and organometallic hydrides can be in particular chosen from the metal catalysts described in the International Patent Applications WO 98/02244, WO 03/061823 and WO 2004/089541, and prepared according to any of the methods described in said Applications.

In stage (1), methane in contact with the catalyst (C1) can react with itself, and produce in particular ethane and hydrogen by a non-oxidative coupling reaction (i.e. a homologation reaction), in particular described according to the following equation (1):

2 CH₄ → C₂H₆ + H₂ (1)

Stage (1) thus leads to formation of a mixture (M1) comprising ethane and hydrogen, and optionally unreacted methane. Under the operating conditions of stage (1), the mixture (M1) is generally obtained in gaseous form.

Stage (1) can be performed under conditions specially suited to promoting the development of the non-oxidative methane coupling reaction towards an optimum production of ethane. Thus, it can be performed at a temperature chosen in a range of from 150 to 700°C, preferably 200 to 650 °C, more particularly 300 to 600 °C. It can also be performed under an absolute total pressure chosen in a range of from 0.1 to 50 MPa, preferably 0.1 to 30 MPa, more particularly 0.1 to 20 MPa. It can also be performed in the presence of at least one inert agent, either liquid or gaseous, in particular of an inert gas chosen from nitrogen, helium and argon.

Stage (1) can be performed in various ways in a reaction zone (Z1), either intermittently, or discontinuously, or preferably continuously. It is possible, for example, to add methane to the catalyst (C1), or to add the catalyst (C1) to methane, or else to add methane and the catalyst (C1) simultaneously into the reaction zone (Z1).

In addition, the reaction zone (Z1) can comprise at least one reactor chosen from static reactors, recycling reactors and dynamic reactors. For example, stage (1) can be performed in a static reactor containing fixed quantities of methane and of catalyst (C1). Stage (1) can also be performed in a recycling reactor into which at least one component of the mixture (M1), for example ethane or optionally unreacted methane can be recycled, preferably continuously, after it has been separated from the mixture (M1) beforehand. Stage (1) can also be performed in a dynamic reactor containing the catalyst (C1), in the form of solid particles, or as a bed or a distillation packing, through which methane can pass or circulate, preferably continuously.

In practice, stage (1) can be performed in a reaction zone (Z1) comprising at least one reactor chosen from tubular (or multi-tubular) reactors, distillation column reactors, slurry reactors, fluidised bed reactors, mechanically agitated bed reactors, fluidised and mechanically agitated bed reactors, fixed bed reactors, moving bed reactors and circulating bed reactors. The catalyst (C1) is generally solid, and can optionally be mixed with an inert solid agent chosen in particular from silicas, aluminas, silica-aluminas and aluminium silicates, either simple or modified by other metals. The catalyst (C1) can be arranged inside the tubes of a tubular (or multi-tubular) reactor. It can also be arranged inside a distillation column reactor, wherein it is preferably a component of a distillation system functioning as both a catalyst and a distillation packing, i.e. a packing for a distillation column having both a distillation function (as in stage (2)) and a catalytic function (as in stage (1)) : for example, rings, saddles, balls, granulates, irregular shapes, granulates, sheets, tubes, spirals, packed in bags, as described in American Patent US 4,242,530. The catalyst (C1) can also form the bed of a fluidised and/or mechanically agitated bed, fixed bed, moving bed or circulating bed of said reactors. Methane can then be introduced in the form of a stream, preferably continuously, into one of said reactors, and in particular pass or circulate into the tubes or through the bed or the distillation packing of said reactors. In order to promote the development of the methane coupling reaction towards an optimum production of ethane, stage (1) can advantageously be performed by withdrawing at least ethane or unreacted methane out of the reaction zone (Z1), preferably continuously.

Thus, for example, stage (1) can be performed:
(i) by continuously introducing methane into the reaction zone (Z1) containing the catalyst (C1), so as to form continuously the mixture (M1) comprising ethane, hydrogen and optionally unreacted methane, and
(ii) by continuously withdrawing at least one part of the mixture (M1) out of the reaction zone (Z1), so as to submit said part of the mixture (M1) optionally to stage (2) for separating ethane from the mixture (M1) and isolating ethane from the rest of the mixture, or directly to stage (3), and preferably to return the rest of the mixture, preferably just the unreacted methane, and optionally the other non-separated part of the mixture (M1) into the reaction zone (Z1), particularly in order to pursue the methane coupling reaction.

The process then optionally comprises a stage (2) comprising separating ethane from the mixture (M1), in order to isolate the ethane. Stage (2) can be performed in various ways, either intermittently, or discontinuously, or preferably continuously. It can comprise one or more methods of fractionation of the mixture (M1), in particular chosen from any known methods or combinations of method for fractionation of such a mixture, preferably from the following six types of fractionation:
- fractionation by change of physical state, preferably of liquid/gaseous phase of the mixture, in particular by distillation or by condensation, preferably by partial condensation, e.g. by means of distillation/condensation column or tower,
- fractionation by molecular filtration, preferably by means of semi-permeable and selective membrane,
- fractionation by adsorption, preferably by means of molecular sieve or any other adsorbent,
- fractionation by absorption, preferably by means of absorbing oil,
- fractionation by cryogenic expansion, preferably by means of expansion turbine, and
- fractionation by compression, preferably by means of gas compressor.

Any type of fractionation that is useful in general for separating ethane from hydrogen and optionally methane, can be utilized in stage (2).

Stage (2) thus makes it possible to separate and isolate ethane and preferably hydrogen and optionally unreacted methane from the mixture (M1). It can be performed in a fractionation zone (F1) which is either separate and distinct from the reaction zone (Z 1) wherein stage (1) is performed, or located in a part of said reaction zone (Z1), preferably in a distillation column reactor as previously mentioned and described in American Patent US 4,242,530.

Thus, a distillation column reactor can be advantageously used both for stage (1) and stage (2), and contain the catalyst (C1). More particularly, the catalyst (C1) can be arranged inside the distillation column reactor, as a component of a distillation system functioning as both a catalyst and a distillation packing. Thus, stages (1) and (2) can be simultaneously performed in at least one distillation column reactor comprising both a reaction zone and a fractionation zone. In the reaction zone, the contacting of the methane with the catalyst (C1) is performed as in stage (1), so as to form the mixture (M1) preferably in a liquid/gaseous phase. Simultaneously, in the fractionation zone, the separation of ethane from the mixture (M1) is performed as in stage (2), preferably by distillation or by condensation, so as to isolate the ethane.

More generally, a fractionation of the mixture (M1) can be performed by change of the physical state, preferably of the liquid/gaseous phase of the mixture, particularly by distillation or by condensation, in particular by partial condensation, e.g. in at least one distillation/condensation column or tower, or in a distillation column reactor. The fractionation can be performed in particular by refrigeration or cryogenic processes, preferably by at least one cooling of the gaseous mixture (M1) to at least one temperature at which at least one component of the mixture condenses and the rest of the mixture remains in gaseous form, so as to separate and to isolate said component(s) from said rest of the mixture.

Generally, a fractionation of the mixture (M1) can also be performed by molecular filtration, preferably by passing the gaseous mixture (M1) through at least one semi-permeable and selective membrane, so as to arrest and to isolate at least one component of the mixture and to allow the rest of the mixture to pass through.

Generally, the fractionation of the mixture (M1) can also be performed by adsorption, preferably by passing the gaseous mixture (M1) over at least one molecular sieve or any other adsorbent, so as to retain at least one component of the mixture and to allow the rest of the mixture to pass through, the component(s) thus retained being then submitted to at least one desorption step, preferably by the TSA method ("Temperature Swing Adsorption") or the PSA method ("Pressure Swing Adsorption"), so as to isolate said component(s).

Generally, a fractionation of the mixture (M1) can also be performed by absorption, in particular by contacting the gaseous mixture (M1) with an absorbing oil, e.g. in an absorption tower, so as to soak up at least one component of the mixture, to form an oil/component mixture and to allow the rest of the gaseous mixture to be free and not absorbed, then by subjecting said oil/component mixture, e.g. in a recovery tower, to a temperature above the boiling point of the component, but below that of the oil, so as to separate and to isolate said component from the oil.

Generally, a fractionation of the mixture (M1) can also be performed by cryogenic expansion, in particular by dropping the temperature of the gaseous mixture (M1) to a temperature so as to condense at least one component of the mixture while maintaining the rest of the mixture in gaseous form, e.g. by means of an expansion turbine which rapidly expands the gaseous mixture and causes a significant drop of the temperature.

Generally, a fractionation of the mixture (M1) can also be performed by compression, in particular by compressing the gaseous mixture (M1) e.g. by means of a gas compressor, so as to condense at least one component of the mixture, to separate and to isolate said component, and to allow the rest of the mixture to be maintained in gaseous form.

For example, a fractionation of the gaseous mixture (M1) can be performed by change of the gaseous phase of the mixture, e.g. in at least one distillation/condensation column or tower, or in a distillation column reactor, preferably by at least one cooling of the gaseous mixture to a temperature at which ethane condenses, so as to condense the ethane, to separate and to isolate it in liquid form from the rest of the gaseous mixture comprising hydrogen and optionally unreacted methane.

For example, a fractionation of the gaseous mixture (M1) can also be performed by molecular filtration of the mixture, preferably by passing the gaseous mixture through at least one semi-permeable and selective membrane, so as to arrest and to isolate the ethane, and to allow the rest of the gaseous mixture comprising hydrogen and optionally unreacted methane, to pass through.

The semi-permeable and selective membranes can be chosen from any those suitable for arresting and separating ethane from hydrogen and optionally methane, preferably chosen from zeolite membranes, porous alumina membranes, ceramic membranes, flowing liquid membranes and supported liquid membranes.

For example, a fractionation of the gaseous mixture (M1) can also be performed by adsorption, preferably by passing the gaseous mixture over at least one molecular sieve or any other adsorbent, so as to retain the ethane, and to allow the rest of the gaseous mixture comprising hydrogen and optionally unreacted methane to pass through, and then to subject the ethane thus retained to a desorption step, for instance performed by the TSA or the PSA method, so as to isolate the ethane.

The adsorbents can be chosen from any those capable of retaining and separating ethane from hydrogen and optionally methane, preferably chosen from molecular sieves, silica gels, activated charcoals and activated carbons.

For example, a fractionation of the gaseous mixture (M1) can also be performed by absorption, preferably by contacting the gaseous mixture (M1) with an absorption oil, e.g. in an absorption tower, so as to soak up ethane from the mixture and to form an oil/ethane mixture, and then preferably by subjecting said mixture, e.g. in a recovery tower, to a temperature above the boiling point of ethane, but below that of the oil, so as to separate and to isolate the ethane from the oil and from the rest of the gaseous mixture comprising hydrogen and optionally unreacted methane.

For example, a fractionation of the gaseous mixture (M1) can also be performed by cryogenic expansion, preferably by dropping the temperature of the gaseous mixture (M1) in particular by use of external refrigerants to cool the gaseous mixture and then by means of an expansion turbine which rapidly expands the chilled gaseous mixture and causes the temperature to drop significantly, so as to condense ethane, to separate and to isolate it from the rest of the gaseous mixture comprising hydrogen and optionally unreacted methane.

For example, a fractionation of the gaseous mixture (M1) can also be performed by compression, preferably by compressing the gaseous mixture (M1), e.g. by means of a gas compressor, so as to condense ethane, to separate and to isolate it in liquid form from the rest of the gaseous mixture comprising hydrogen and optionally unreacted methane.

Stage (2) can be performed advantageously by a double (or multiple) fractionation of the mixture (M1) comprising a combination of two (or more) successive fractionations of an identical or different type, chosen from the six above-mentioned types of fractionation, so as in particular to separate and to isolate (a) ethane, (b) hydrogen and (c) unreacted methane.

The unreacted methane can be advantageously separated and isolated in stage (2) and then recycled into stage (1) for methane coupling. Moreover, it may also be particularly advantageous to separate and to isolate hydrogen from the mixture (M1), and then to use it in one or more applications or stages, distinct or not from the present process. For example, the hydrogen thus isolated can be used as an agent for activation or regeneration of the catalyst(s) (C1) and/or (C2) used in the process. The activation or the regeneration of the catalyst can be carried out by contacting the catalyst with the hydrogen, during one of the stages of the process, for example during stage (3) for ethane metathesis, or in a stage distinct and separate from the process. The hydrogen can also be used highly advantageously in other applications or stages, distinct or not from the present process, for example as a fuel for producing thermal and/or electrical energies, in particular as a fuel in hydrogen fuel cells for producing electrical energy, such energies being preferably for running the present process, or as a fuel for automobile, or as a reagent in a chemical, petrochemical or refinery plant. IT is remarkable to notice that hydrogen produced and isolated by the process can generally supply all the energy needed to run said process.

For example, it is possible to perform a double fractionation by subjecting the gaseous mixture (M1) comprising ethane, hydrogen and unreacted methane, to a combination of two successive fractionations of one and the same type, namely by change of physical state, preferably of the liquid/gaseous phase of the mixture, e.g. in one or more distillation/condensation columns or towers, or in a distillation column reactor, for example as follows:
(i) by a first cooling of the gaseous mixture (M1) to a temperature at which ethane condenses, so as to condense the ethane, to separate and to isolate it in liquid form from the rest of the gaseous mixture comprising hydrogen and unreacted methane, and
(ii) by a second cooling of said rest of the gaseous mixture to a temperature at which methane condenses, so as to condense the unreacted methane, to separate and to isolate it in liquid form from hydrogen which is preferably isolated in its turn in gaseous form.

It is also possible to perform a double fractionation by subjecting the above-mentioned gaseous mixture (M1) to a combination of two successive fractionations of one and the same type, namely by molecular filtration:
(i) by passing the gaseous mixture (M1) through a first semi-permeable and selective membrane, so as to arrest and to isolate the ethane, and to allow the rest of the gaseous mixture comprising hydrogen and unreacted methane to pass through, and
(ii) by passing said rest of the gaseous mixture through a second semi-permeable and selective membrane, so as to arrest and to isolate the methane, and to allow hydrogen to pass through and preferably to be isolated in its turn in gaseous form.

It is also possible to perform a double fractionation by subjecting the above-mentioned gaseous mixture (M1) to a combination of two successive fractionations of one and the same type, namely by adsorption:
(i) by passing the gaseous mixture (M1) onto a first molecular sieve or any other adsorbent, so as to retain ethane and to allow the rest of the gaseous mixture comprising hydrogen and unreacted methane to pass through, then
(ii) by passing said rest of the gaseous mixture onto a second molecular sieve or any other adsorbent, so as to retain unreacted methane (or hydrogen), and to allow the hydrogen (or the unreacted methane) to pass through, and
(iii) by respectively submitting ethane and methane (or hydrogen) thus retained to desorption steps, preferably performed by the TSA or the PSA method, so as respectively to isolate the ethane and the unreacted methane (or the hydrogen).

For example, it is also possible to perform a double fractionation by subjecting the above-mentioned gaseous mixture (M1) to a combination of two successive fractionations of one and the same type, namely by absorption, e.g. in one or more absorption towers:
(i) by contacting the gaseous mixture (M1) with a first absorbing oil, so as to soak up ethane from the mixture, to form an oil/ethane mixture, and to allow the rest of the gaseous mixture comprising unreacted methane and hydrogen to be free in gaseous form,
(ii) by contacting said rest of the gaseous mixture with a second absorbing oil, so as to soak up methane, to form an oil/methane mixture, and to allow hydrogen to be free in gaseous form and preferably to be isolated, and
(iii) by subjecting said oil/ethane mixture to a temperature above the boiling point of ethane, but below that of the oil, e.g. in a recovery tower, so as to separate and to isolate the ethane from the oil, and preferably by subjecting said oil/methane mixture to a temperature above the boiling point of methane, but below that of the oil, e.g. in a recovery tower, so as to separate and to isolate the unreacted methane from the oil.

For example, it is also possible to perform a double fractionation by subjecting the above-mentioned gaseous mixture (M1) to a combination of two successive fractionations of one and the same type, namely by cryogenic expansion:
(i) by dropping the temperature of the gaseous mixture (M1) by means of an expansion turbine, so as to condense ethane, to separate and to isolate it in liquid form from the rest of the gaseous mixture comprising hydrogen and unreacted methane, and
(ii) by dropping the temperature of said rest of the gaseous mixture by means of an expansion turbine, so as to condense methane, to separate and to isolate it in liquid form from hydrogen which is preferably isolated in its turn in gaseous form.

For example, it is also possible to perform a double fractionation by subjecting the above-mentioned gaseous mixture (M1) to a combination of two successive fractionations of one and the same type, namely by compression:
(i) by compressing the gaseous mixture (M1), e.g. by means of a gas compressor, so as to condense ethane, to separate and to isolate it in liquid form from the rest of the gaseous mixture comprising unreacted methane and hydrogen, and
(ii) by compressing said rest of the gaseous mixture, e.g. by means of a gas compressor, so as to condense methane, to separate and to isolate it in liquid form from hydrogen which is preferably isolated in its turn in gaseous form.

It is also possible to perform a double (or multiple) fractionation by subjecting the gaseous mixture (M1) comprising ethane, hydrogen and unreacted methane, to a combination of two (or more) successive fractionations of a different type, chosen from the six above-mentioned types of fractionation.

A double fractioning of the above-mentioned gaseous mixture (M1) can, for example, be performed by combining two successive fractionations of a different type, namely:
- the one by change of physical state, preferably of liquid/gaseous phase of the mixture, such as by distillation or by condensation, and the other by molecular filtration, preferably by means of a semi-permeable and selective membrane, or
- the one by change of physical state, preferably of liquid/gaseous phase of the mixture, such as by distillation or by condensation, and the other by adsorption, preferably by means of a molecular sieve or any other adsorbent, or
- the one by change of physical state, preferably of liquid/gaseous phase of the mixture, such as by distillation or by condensation, and the other by absorption, preferably by means of an absorbing oil, or
- the one by change of physical state, preferably of liquid/gaseous phase of the mixture, such as by distillation or by condensation, and the other by cryogenic expansion, preferably by means of an expansion turbine, or
- the one by change of physical state, preferably of liquid/gaseous phase of the mixture, such as by distillation or by condensation, and the other by compression, preferably by means of a gas compressor, or
- the one by molecular filtration, preferably by means of a semi-permeable and selective membrane, and the other by adsorption, preferably by means of a molecular sieve or any other adsorbent, or
- the one by molecular filtration, preferably by means of a semi-permeable and selective membrane, and the other by absorption, preferably by means of an absorbing oil, or
- the one by molecular filtration, preferably by means of a semi-permeable and selective membrane, and the other by cryogenic expansion, preferably by means of an expansion turbine, or
- the one by molecular filtration, preferably by means of a semi-permeable and selective membrane, and the other by compression, preferably by means of a gas compressor, or
- the one by adsorption, preferably by means of molecular sieve or any other adsorbent, and the other by absorption, preferably by means of an absorbing oil, or
- the one by adsorption, preferably by means of molecular sieve or any other adsorbent, and the other by cryogenic expansion, preferably by means of an expansion turbine, or
- the one by adsorption, preferably by means of molecular sieve or any other adsorbent, and the other by compression, preferably by means of a gas compressor, or
- the one by absorption, preferably by means of an absorbing oil, and the other by cryogenic expansion, preferably by means of an expansion turbine, or
- the one by absorption, preferably by means of an absorbing oil, and the other by compression, preferably by means of a gas compressor, or
- the one by cryogenic expansion, preferably by means of an expansion turbine, and the other by compression , preferably by means of a gas compressor.

Each of said combinations can be performed in a chronological order or in a reverse order.

Thus, for example, the two successive fractionations of a different type can be performed advantageously:
(i) by cooling the gaseous mixture (M1) to a temperature at which ethane condenses, so as to condense the ethane, to separate and to isolate it in liquid form from the rest of the gaseous mixture comprising hydrogen and unreacted methane, and
(ii) by passing said rest of the gaseous mixture through a semi-permeable and selective membrane, so as to arrest and to isolate the unreacted methane, and to allow hydrogen to pass through and preferably to be isolated in its turn in gaseous form.

It is also possible, for example, to perform the two above-mentioned successive fractionations in a reverse order, that is to say:
(i) by passing the gaseous mixture (M1) through a semi-permeable and selective membrane, so as to arrest and to isolate ethane and to allow the rest of the gaseous mixture comprising hydrogen and unreacted methane to pass through, and
(ii) by cooling said rest of the gaseous mixture to a temperature at which methane condenses, so as to condense the unreacted methane, to separate and to isolate it in liquid form from hydrogen which is preferably isolated in its turn in gaseous form.

Thus, e.g. by means of a double (or multiple) fractionation such as one of those described above, the methane thus isolated in stage (2) is preferably recycled into stage (1) for non-oxidative methane coupling. Furthermore, the hydrogen thus isolated in stage (2) can be used advantageously in one of the above-mentioned applications, such as an agent of activation or regeneration of the catalyst(s) (C1) and/or (C2), or as a fuel for producing thermal and/or electrical energies, in particular as a fuel in hydrogen fuel cells for producing electrical energy, such energies being preferably employed to run the present process, or as a fuel for automobile, or as a reagent in a chemical, petrochemical or refinery plant.

The process then comprises a stage (3) for ethane metathesis. Stage (3) comprises contacting the ethane isolated in stage (2) or the mixture (M1) obtained in stage (1) with a metal catalyst (C2) capable of producing, in contact with alkane, reactions involving the splitting and re-combining of carbon-carbon and/or carbon-hydrogen and/or carbon-metal bonds, so as to form a mixture (M2) comprising higher alkanes (C₃₊), preferably (C₄₊), methane and optionally unreacted ethane and where applicable propane (in particular when the higher alkanes (C₄₊) are preferably prepared, instead of the higher alkanes (C₃₊)).

The catalyst (C2) can be identical to or preferably different from the catalyst (C1) used in stage (1) for non-oxidative methane coupling, and can conform in particular to the general definition of metal catalysts given above for the catalyst (C1). In the case when the catalyst (C2) is different from catalyst (C1), it might be possible to use directly in stage (3) the mixture (M1) obtained in stage (1), instead of using the ethane isolated in stage (2).

In particular, the catalyst (C2) can be chosen from metal catalysts that are capable of alkane metathesis. It can be chosen from supported metal clusters, and preferably from metal hydrides, organometallic compounds and organometallic hydrides, particularly supported on, or more specifically grafted onto, a solid support.

The catalyst (C2) can be chosen from supported metal clusters comprising one or more metals of Groups 4, 5 and/or 6 of the Periodic Table of the Elements, preferably tantalum. The supported metal clusters generally comprise a solid support and a metal cluster, and preferably comprise bonding between the metals of the metal cluster, and also bonding between the metal cluster and the solid support. Thus, the metal atom of the metal cluster can be bonded to at least one other metal atom of the metal cluster, preferably to six or fewer metal atoms of the metal cluster. The metal atom of the metal cluster can be bonded in addition to at least one hydrocarbon radical and/or to at least one hydrogen atom. The solid support can be chosen from a metal oxide, a refractory oxide, a molecular sieve, a zeolite, a metal phosphate and a material comprising a metal and oxygen, preferably silica. More particularly, the supported metal clusters can comprise tantalum (as a metal cluster) and silica (as a solid support), and preferably comprise bonding between tantalum and tantalum, and also bonding between tantalum and silica, in particular bonding between tantalum and oxygen on the silica surface. The catalyst (C2) can be chosen from the supported metal clusters described in International Patent Application WO 2006/060692, and prepared according to any methods described in said Application.

The catalyst (C2) is preferably chosen from metal hydrides, organometallic compounds and organometallic hydrides, particularly supported on, more specifically grafted onto, a solid support. It can comprise at least one metal chosen from scandium, yttrium, lanthanum, titanium, zirconium, hafnium, vanadium, niobium, tantalum, chromium, molybdenum, tungsten, rhenium, ruthenium, osmium, nickel, iridium, palladium, platinum, cerium and neodymium. The metal of the catalyst can be chosen, preferably, from yttrium, titanium, zirconium, hafnium, vanadium, niobium, tantalum, chromium, molybdenum, tungsten, rhenium, ruthenium and platinum, and more especially chosen from niobium, tantalum, molybdenum, tungsten and rhenium.

The catalyst (C2) can be chosen from metal hydrides, and preferably from organometallic compounds and organometallic hydrides, comprising more particularly at least one hydrocarbon radical (R), either saturated or unsaturated, linear or branched, having preferably from 1 to 20, more particularly from 1 to 14 carbon atoms. The hydrocarbon radicals (R) can be chosen from alkyl radicals, more particularly either saturated or unsaturated, linear or branched, aliphatic or alicyclic, for example alkyl, alkylidene or alkylidyne radicals, more particularly from C₁ to C₁₀, from aryl radicals, more particularly from C₆ to C₁₂, and from aralkyl, aralkylidene or aralkylidyne radicals, more particularly from C₇ to C₁₄. Thus, the metal atom of the catalyst (C2) is preferably bonded to at least one hydrogen atom and/or to at least one hydrocarbon radical (R), in particular by a single, double or triple carbon-metal bond.

The catalyst (C2) preferably selected from metal hydrides, organometallic compounds and organometallic hydrides, can be furthermore modified by one or more ligands, such as "ancillary" ligands, preferably comprising at least one oxygen atom and/or at least one nitrogen atom, and particularly chosen from oxo, alkoxo, aryloxo, aralkyloxo, nitrido, imido and amido ligands. Generally, by oxo, alkoxo, aryloxo, aralkyloxo, nitrido, imido and amido ligands are respectively meant:
- a divalent oxo radical of general formula: = 0
- a monovalent alkoxo, aryloxo or aralkyloxo radical of general formula: - OR'
- a trivalent nitrido radical of general formula: ≡ N
- a divalent imido radical of general formula : = NR", and
- a monovalent amido radical of general formula : - NR¹R² , general formulae in which O represents an oxygen atom, N represents a nitrogen atom, R' represents an hydrogen atom or a monovalent hydrocarbon radical selected respectively from alkyl, aryl and aralkyl radicals, R" represents a n hydrogen atom or a monovalent hydrocarbon radical, and R¹ and R², being identical or different, represent an hydrogen atom or a monovalent hydrocarbon radical. More particularly, R', R", R¹ and R² can be a monovalent hydrocarbon radical, either saturated or unsaturated, linear or branched, comprising from 1 to 20, preferably from 1 to 14 carbon atoms, and particularly chosen from alkyl radicals, preferably from C₁ to C₁₀, aralkyl radicals, preferably from C₇ to C₁₄, and aryl radicals, preferably from C₆ to C₁₂.

Thus, when the catalyst (C2) chosen from metal hydrides, organometallic compounds and organometallic hydrides comprises at least one of the above-mentioned ligands, the metal of the catalyst is not only bonded to at least one hydrogen atom and/or to at least one hydrocarbon radical (R), but also to at least one of these ligands, e.g. to the O atom of the oxo radical by a double bond, or to the O atom of the alkoxo, aryloxo or aralkyloxo radical (OR') by a single bond, or to the N atom of the nitrido radical by a triple bond, or to the N atom of the imido radical (NR") by a double bond, or to the N atom of the amido radical (NR¹R²) by a single bond. Generally, the presence of the oxo, alkoxo, aryloxo, aralkyloxo, nitrido, imido and/or amido ligands in the catalyst (C2) favourably influences the behaviour of the catalyst in the ethane metathesis reactions of stage (3).

In addition, the catalyst (C2) chosen from metal hydrides, organometallic compounds and organometallic hydrides is preferably supported on, or more particularly grafted onto, a solid support that can be chosen from metal or refractory oxides, sulfides, carbides, nitrides and salts, and from carbon, mesoporous materials, organic/inorganic hybrid materials, metals and molecular sieves. The solid support can be chosen from silicas, aluminas, silica-aluminas and aluminium silicates, either simple or modified by other metals, and from zeolites, clays, titanium oxide, cerium oxide, magnesium oxide, niobium oxide, tantalum oxide and zirconium oxide. It can be chosen, preferably, from silicas, aluminas, silica-aluminas and aluminium silicates, either simple or modified by other metals. The catalyst (C2) is preferably a metal hydride or an organometallic hydride grafted onto a solid support, more specifically chosen from niobium hydrides or organometallic niobium hydrides grafted onto silica, tantalum hydrides or organometallic tantalum hydrides grafted onto silica, molybdenum hydrides or organometallic molybdenum hydrides grafted onto silica, tungsten hydrides or organometallic tungsten hydrides grafted onto alumina, and rhenium hydrides or organometallic rhenium hydrides grafted onto silica.

The catalyst (C2) chosen from metal hydrides, organometallic compounds and organometallic hydrides can be in particular chosen from the metal catalysts described in International Patent Applications WO 98/02244, WO 03/061823 and WO 2004/089541, and prepared according to any of the methods described in said Applications.

In stage (3), it is observed that ethane can react with itself in contact with the catalyst (C2), and produce more particularly methane and propane by a self-metathesis reaction, in particular according to the following equation (2):

2 C₂H₆ → CH₄ + C₃H₈ (2)

It is observed furthermore that stage (3) can involve a metathesis reaction of ethane with the propane obtained previously, and produce especially methane and butanes, in particular according to the following equation (3):

C₂H₆ + C₃H₈ → CH₄ + C₄H₁₀ (3)

It is also observed that stage (3) can also involve other similar metathesis reactions of ethane, in particular between ethane and successively higher alkanes having at least 4 carbon atoms, such as obtained by other preceding metathesis reactions of ethane, and produce in particular methane and higher alkanes having one additional carbon atom compared with the higher alkanes involved in the reactions, for example according to the following equations (4) to (7):

C₂H₆ + C₄H₁₀ → CH₄ + C₅H₁₂ (4)

C₂H₆ + C₅H₁₂ → CH₄ + C₆H₁₄ (5)

C₂H₆ + C₆H₁₄ → CH₄ + C₇H₁₆ (6)

C₂H₆ + C₇H₁₆ → CH₄ + C₈H₁₈ (7)

It is thus found that in the process of the invention, ethane appears to be an essential intermediate alkane which allows ultimately for methane to be easily converted into higher alkanes (C₃₊), preferably (C₄₊). It is remarkable to note that the conversion can be carried out with a particularly high overall yield, particularly when the methane produced in stage (3) is separated and isolated from the mixture (M2) and then recycled into stage (1) for methane coupling. Metathesis reactions between (C₃₊) or (C₄₊) alkanes and other (C₃₊) or (C₄₊) alkanes are also possible in stage (3) leading to the formation of higher alkanes.

Stage (3) leads to formation of a mixture (M2) comprising higher alkanes (C₃₊), preferably (C₄₊), methane, and optionally unreacted ethane and where applicable propane. Under the operating conditions of stage (3), the mixture (M2) is generally obtained in gaseous form or in mixed gaseous/liquid form.

Stage (3) can advantageously be performed in conditions suited to promoting the development of the ethane metathesis reactions such as described above, in particular towards an optimum production of higher alkanes (C₃₊), preferably (C₄₊). Thus, it can be performed at a temperature chosen in a range of from 20 to 400°C, preferably 50 to 350 °C, particularly 70 to 300 °C, more particularly 100 to 250 °C. It can also be performed under an absolute total pressure chosen in a range of from 0.1 to 50 MPa, preferably 0.1 to 30 MPa, particularly 0.1 to 20 MPa. It can also be performed in the presence of at least one inert agent, either liquid or gaseous, in particular of at least one inert gas chosen from nitrogen, helium and argon. It can also be performed in the presence of hydrogen, preferably of the hydrogen separated from the mixture (M1) in stage (2), in particular under a partial pressure of hydrogen chosen in a range of from 0.0001 to 10 MPa, preferably from 0.001 to 1 MPa.

Stage (3) can be performed in various ways, in a reaction zone (Z2) which can be either distinct and separate from the reaction zone (Z1) or not, either intermittently, or discontinuously, or preferably continuously. It is possible, for example, to add ethane (or the mixture (M1)) to the catalyst (C2), or to add the catalyst (C2) to ethane (or to the mixture (M1)), or else to add ethane (or the mixture (M1)) and the catalyst (C2) simultaneously into the reaction zone (Z2).

The reaction zone (Z2) can be of a type identical to or different from that of the reaction zone (Z1) of stage (1), as described above. Thus, the reaction zone (Z2) can comprise at least one reactor chosen from static reactors, recycling reactors and dynamic reactors. For example, stage (3) can be performed in a static reactor containing fixed quantities of ethane (or of the mixture (M1)) and of catalyst (C2). Stage (3) can also be performed in a recycling reactor into which at least one component of the mixture (M2), for example methane, higher alkanes (C₃₊) or (C₄₊) and optionally unreacted ethane and where applicable propane, can be recycled, preferably continuously, after it has been preferably separated from the mixture (M2) beforehand. Stage (3) can also be performed in a dynamic reactor containing the catalyst (C2), in the form of solid particles, or as a bed or a distillation packing, through which ethane can pass or circulate, preferably continuously.

In practice, stage (3) can be performed in a reaction zone (Z2) comprising at least one reactor chosen from tubular (or multi-tubular) reactors, distillation column reactors, slurry reactors, fluidised bed reactors, mechanically agitated bed reactors, fluidised and mechanically agitated bed reactors, fixed bed reactors, moving bed reactors and circulating bed reactors. The catalyst (C2) is generally solid, and can optionally be mixed with an inert solid agent chosen in particular from silicas, aluminas, silica-aluminas and aluminium silicates, either simple or modified by other metals. The catalyst (C2) can be arranged inside the tubes of a tubular (or multi-tubular) reactor. It can also be arranged inside a distillation column reactor, wherein it is preferably a component of a distillation system functioning as both a catalyst and a distillation packing, i.e. a packing for a distillation column having both a distillation function (as in stage (4)) and a catalytic function (as in stage (3): for example, rings, saddles, balls, granulates, irregular shapes, granulates, sheets, tubes, spirals, packed in bags, as described in American Patent US 4,242,530. The catalyst (C2) can form the bed of a fluidised and/or mechanically agitated bed, fixed bed, moving bed or circulating bed of said reactors. Ethane (or the mixture (M1)) can then be introduced in the form of a stream, preferably continuously, into one of said reactors, and pass or circulate into the tubes or through the beds or the distillation packing of said reactors. In order to promote the development of the ethane metathesis reactions towards an optimum production of higher alkanes (C₃₊), preferably (C₄₊), stage (3) can advantageously be performed by withdrawing the higher alkanes (C₃₊), preferably (C₄₊) and/or methane out of the reaction zone (Z2), preferably continuously.

In practice, metathesis stage (3) can be performed:
(i) by continuously introducing ethane (or the mixture (M1)) into the reaction zone (Z2) containing the catalyst (C2), so as to form the mixture (M2) comprising the higher alkanes (C₃+), preferably (C₄+), methane and optionally unreacted ethane and where applicable propane, and
(ii) by continuously withdrawing at least one part of the mixture (M2) out of the reaction zone (Z2), so as to subject said part of the mixture (M2) to stage (4) for separation of the higher alkanes (C₃₊), preferably (C₄₊) from the rest of the mixture, and preferably to return said rest of the mixture, particularly the unreacted ethane and where applicable the propane, and optionally the other non-separated part of the mixture (M2) into the reaction zone (Z2), in order to continue the ethane metathesis.

The process then comprises a stage (4) comprising separating the higher alkanes (C₃₊), preferably (C₄₊) from the mixture (M2), so as to isolate said higher alkanes. Stage (4) can be performed in various ways, either intermittently, or discontinuously, or preferably continuously. It can comprise one or more methods of fractionation of the mixture (M2), in particular chosen from any known methods or combinations of method for fractionation of such a mixture, preferably from the following six types of fractionation:
- fractionation by change of physical state, preferably by change of liquid/gaseous phase of the mixture, in particular by distillation or by condensation, particularly by partial condensation, in particular by means of distillation/condensation column or tower,
- fractionation by molecular filtration, preferably by means of semi-permeable and selective membrane,
- fractionation by adsorption, preferably by means of molecular sieve or any other adsorbent,
- fractionation by absorption, preferably by means of absorbing oil,
- fractionation by cryogenic expansion, preferably by means of expansion turbine, and
- fractionation by compression, preferably by means of gas compressor.

Any method that is useful in general for separating the higher alkanes (C₃₊), preferably (C₄₊) from methane and optionally ethane and where applicable propane, can be utilized in stage (4).

Stage (4) thus makes it possible to separate higher alkanes (C₃₊), preferably (C₄₊) from the mixture (M2) and isolate them. It can also permit methane to be separated and isolated from the mixture (M2), and then preferably recycled into stage (1) for non-oxidative methane coupling. In addition, it can permit that unreacted ethane and/or where applicable propane is/are separated and isolated from the mixture (M2) in stage (4), and then preferably is/are recycled into stage (3) for ethane metathesis, in particular together or separately.

Stage (4) can be performed in a fractionation zone (F2) which is either distinct and separate from the reaction zone (Z2) wherein stage (3) is performed, or located in a part of said reaction zone (Z2), preferably in a distillation column reactor, as previously mentioned and described in American Patent US 4,242,530. Stage (4) may also be carried out in an integral part of stage (3).

Thus, the distillation column reactor can be advantageously used both for stage (3) and stage (4), and contain the catalyst (C2). More particularly, the catalyst (C2) can be arranged inside the distillation column reactor, as a component of a distillation system functioning both as a catalyst and a distillation packing. Thus, stages (3) and (4) can be simultaneously performed in at least one distillation column reactor comprising both a reaction zone and a fractionation zone. In the reaction zone, the contacting of the ethane (or of the mixture (M1)) with the catalyst (C2) can be performed as in stage (3), so as to form the mixture (M2) preferably in liquid/gaseous phase. Simultaneously, in the fraction zone, the separation of the higher alkanes (C₃₊), preferably (C₄₊) from the mixture (M2) can be performed as in stage (4), preferably by distillation/condensation, so as to isolate said higher alkanes.

More generally, a fractionation of the mixture (M2) can be performed by change of the physical state, preferably of the liquid/gaseous phase of the mixture, particularly by distillation or by condensation (or liquefaction), more particularly by partial condensation (or partial liquefaction), e.g. in at least one distillation/ condensation column or tower, or in a distillation column reactor. The fractionation can be performed by refrigeration or cryogenic processes, in particular by at least one cooling of the mixture (M2) to at least one temperature at which at least one component of the mixture condenses and the rest of the mixture remains in gaseous form.

Generally, a fractionation of the mixture (M2) can also be performed by molecular filtration, preferably by passing the mixture (M2) through at least one semi-permeable and selective membrane, so as to arrest and to isolate at least one component of the mixture and to allow the rest of the mixture to pass through.

Generally, a fractionation of the mixture (M2) can also be performed by adsorption, preferably by passing the mixture (M2) over at least one molecular sieve or any other adsorbent, so as to retain at least one component of the mixture and to allow the rest of the mixture to pass through, and then by subjecting the component(s) thus retained to at least one desorption step, preferably by the TSA or the PSA method, so as to isolate said component(s).

Generally, a fractionation of the mixture (M2) can also be performed by absorption, preferably by contacting the mixture (M2) with an absorbing oil, so as to soak up at least one component of the mixture, to form an oil/component mixture and to allow the rest of the mixture to be free and not absorbed, and then by subjecting said oil/component mixture to a temperature above the boiling point of the component(s), but below that of the oil, so as to separate and to isolate said component(s) from the oil.

Generally, a fractionation of the mixture (M2) can also be performed by cryogenic expansion, preferably by dropping the temperature of the mixture (M2) to a temperature so as to condense at least one component of the mixture while maintaining the rest of the mixture in unchanged form, e.g. by means of an expansion turbine which rapidly expands the mixture and causes a significant drop of its temperature.

Generally, a fractionation of the mixture (M2) can also be performed by compression, preferably by compressing the mixture (M2), e.g. by means of a gas compressor, so as to condense at least one component of the mixture, to separate and to isolate it from the rest of the gaseous mixture.

For example, a fractionation of the gaseous mixture (M2) can be performed by change of physical state, preferably of the liquid/gaseous phase of the mixture, e.g. in at least one distillation/condensation column or tower, or in a distillation column reactor, in particular by at least one cooling of the gaseous mixture to a temperature at which the higher alkanes (C₃₊), preferably (C₄₊) condense, so as to condense said higher alkanes, to separate and to isolate them in liquid form from the rest of the gaseous mixture comprising methane and optionally unreacted ethane and where applicable propane.

For example, a fractionation of the gaseous mixture (M2) can also be performed by molecular filtration of the mixture, in particular by passing the gaseous mixture through at least one semi-permeable and selective membrane, so as to arrest and to isolate the higher alkanes (C₃₊), preferably (C₄₊), and to allow the rest of the gaseous mixture comprising methane and optionally unreacted ethane and where applicable propane to pass through.

The semi-permeable and selective membranes can be chosen from any membrane suitable for arresting and separating the higher alkanes (C₃₊), preferably (C₄₊) from methane, optionally ethane and where applicable propane. They can be preferably chosen from zeolite membranes, porous alumina membranes, ceramic membranes, flowing liquid membranes and supported liquid membranes.

For example, a fractionation of the gaseous mixture (M2) can also be performed by adsorption, in particular by passing the gaseous mixture through at least one molecular sieve or any other adsorbent, so as to retain the higher alkanes (C₃₊), preferably (C₄₊), and to allow the rest of the gaseous mixture comprising methane and optionally unreacted ethane and where applicable propane to pass through, and then by subjecting said higher alkanes thus retained to at least one desorption step, for instance performed by the TSA or the PSA method, so as to isolate them.

The adsorbents can be chosen from any adsorbent suitable for retaining and separating the higher alkanes (C₃₊), preferably (C₄₊) from methane, optionally ethane and where applicable propane. They can be preferably chosen from molecular sieves, silica gels, activated charcoals and activated carbons.

For example, a fractionation of the gaseous mixture (M2) can also be performed by absorption, preferably by contacting the gaseous mixture (M2) with an absorption oil, e.g. in an absorption tower, so as to soak up the higher alkanes (C₃₊), preferably (C₄₊), and to form an oil/higher alkane (C₃₊) or (C₄₊) mixture, and then preferably by subjecting said oil/higher alkane (C₃₊) or (C₄₊) mixture, e.g. in a recovery tower, to a temperature above the boiling point of said higher alkanes, but below that of the oil, so as to separate and to isolate said higher alkanes from the oil and from the rest of the gaseous mixture comprising methane and optionally unreacted ethane and where applicable propane.

For example, a fractionation of the gaseous mixture (M2) can also be performed by cryogenic expansion, preferably by dropping the temperature of the gaseous mixture (M2) in particular by use of external refrigerants to cool the gaseous mixture and then by means of an expansion turbine which rapidly expands the chilled gaseous mixture and causes the temperature to drop significantly, so as to condense the higher alkanes (C₃+), preferably (C₄+), to separate and to isolate them in liquid form from the rest of the gaseous mixture comprising methane and optionally unreacted ethane and where applicable propane.

For example, a fractionation of the gaseous mixture (M2) can also be performed by compression, preferably by compressing the gaseous mixture (M2), e.g. by means of a gas compressor, so as to condense the higher alkanes (C₃₊), preferably (C₄₊), to separate and to isolate them in liquid form from the rest of the gaseous mixture comprising methane and optionally unreacted ethane and where applicable propane.

Stage (4) can be performed advantageously by a double (or multiple) fractionation of the mixture (M2), comprising a combination of two (or more) successive fractionations of an identical or different type, chosen from the six above-mentioned types of fractionation, so as in particular to separate and to isolate (a) the higher alkanes (C₃₊), preferably (C₄₊), (b) methane and (c) unreacted ethane and where applicable propane.

Thus, methane can be advantageously separated and isolated in stage (4), and then recycled into stage (1) for non-oxidative methane coupling. In the same way, unreacted ethane and/or where applicable propane is/are also preferably separated and optionally isolated in stage (4), and then preferably is/are recycled into stage (3) for ethane metathesis, in particular together or separately. The separation, isolation and recycling of the methane are particularly advantageous in the process of the invention, since methane is produced in large quantities in stage (3), and its recycling into stage (1) for methane coupling improves enormously the overall yield of the process. The same applies to the separation, isolation and recycling of unreacted ethane and where applicable of propane, since it (or their) recycling into stage (3) for ethane metathesis improves substantially the output of said stage.

For example, a double fractionation can be performed by subjecting the gaseous mixture (M2) comprising the higher alkanes (C₃₊), preferably (C₄₊), methane and unreacted ethane and where applicable propane, to a combination of two successive fractionations of one and the same type, namely by change of physical state, preferably of the liquid/gaseous phase of the mixture, e.g. in one or more distillation/condensation columns or towers, or in a distillation column reactor:
(i) by a first cooling of the gaseous mixture (M2) to a temperature at which the higher alkanes (C₃₊), preferably (C₄₊) condense, so as to condense said higher alkanes, and to separate and to isolate them in liquid form from the rest of the gaseous mixture comprising methane, unreacted ethane and where applicable propane, and
(ii) by a second cooling of said rest of the gaseous mixture to a temperature at which ethane and where applicable propane condense(s), so as to condense the ethane and where applicable the propane, and to separate and to isolate it (or them) in liquid form from methane which is preferably isolated in its turn in gaseous form.

In practice, a double fractionation can also be performed by subjecting the above-mentioned gaseous mixture (M2) to a combination of two successive fractionations of one and the same type, namely by molecular filtration:
(i) by passing the gaseous mixture (M2) through a first semi-permeable and selective membrane, so as to arrest and to isolate the higher alkanes (C₃₊), preferably (C₄₊), and to allow the rest of the gaseous mixture comprising methane, unreacted ethane and where applicable propane to pass through, and
(ii) by passing said rest of the gaseous mixture through a second semi-permeable and selective membrane, so as to arrest and to isolate the ethane and where applicable the propane, and to allow methane to pass through and preferably to be isolated in its turn in gaseous phase.

In practice, a double fractionation can also be performed by subjecting the above-mentioned gaseous mixture (M2) to a combination of two successive fractionations of one and the same type, namely by adsorption:
(i) by passing the gaseous mixture (M2) onto a first molecular sieve or any other adsorbent, so as to retain the higher alkanes (C₃₊), preferably (C₄₊), and to allow the rest of the gaseous mixture comprising methane, unreacted ethane and where applicable propane to pass through, then
(ii) by passing said rest of the gaseous mixture onto a second molecular sieve or any other adsorbent, so as to retain the unreacted ethane and where applicable the propane, and to allow methane) to pass through and preferably to be isolated in gaseous form, and
(iii) by respectively submitting said higher alkanes and the unreacted ethane and where applicable the propane thus retained to desorption steps, in particular performed by the TSA or PSA method, so as respectively to isolate said higher alkanes and the unreacted ethane and where applicable the propane.

In practice, it is also possible to perform a double fractionation by subjecting the above-mentioned gaseous mixture (M2) to a combination of two successive fractionations of one and the same type, namely by absorption, e.g. in one or more absorption towers:
- (i) by contacting the gaseous mixture (M2) with a first absorbing oil, so as to soak up the higher alkanes (C₃₊), preferably (C₄₊), to form an oil/higher alkane (C₃₊) or (C₄₊) mixture, and to allow the rest of the gaseous mixture comprising methane, unreacted ethane and where applicable propane to be free in gaseous form,
- (ii) by contacting said rest of the gaseous mixture with a second absorbing oil, so as to soak up the unreacted ethane and where applicable the propane, to form an oil/ethane/(propane) mixture, and to allow methane to be free in gaseous form and preferably to be separated and isolated, and
- (iii) by subjecting said oil/higher alkane (C₃₊) or (C₄₊) mixture to a temperature above the boiling point of said higher alkanes, but below that of the oil, e.g. in a recovery tower, so as to separate and to isolate said higher alkanes from the oil, and preferably by subjecting said oil/ethane/(propane) mixture to a temperature above the boiling point of ethane (and where applicable of propane), but below that of the oil, e.g. in a recovery tower, so as to separate and to isolate the unreacted ethane (and where applicable the propane) from the oil.

In practice, it is also possible to perform a double fractionation by subjecting the above-mentioned gaseous mixture (M2) to a combination of two successive fractionations of one and the same type, namely by cryogenic expansion:
- (i) by dropping the temperature of the gaseous mixture (M2) by means of an expansion turbine, so as to condense the higher alkanes (C₃₊), preferably (C₄₊), and to separate and to isolate them in liquid form from the rest of the gaseous mixture comprising methane, unreacted ethane and where applicable propane, and
- (ii) by dropping the temperature of said rest of the gaseous mixture by means of an expansion turbine, so as to condense the unreacted ethane (and where applicable the propane), to separate and to isolate it (or them) in liquid form from methane which is preferably isolated in its turn in gaseous form.

In practice, it is also possible to perform a double fractionation by subjecting the above-mentioned gaseous mixture (M2) to a combination of two successive fractionations of one and the same type, namely by compression:
- (i) by compressing the gaseous mixture (M2), e.g. by means of a gas compressor, so as to condense the higher alkanes (C₃₊), preferably (C₄₊), to separate and to isolate them in liquid form from the rest of the gaseous mixture comprising methane, unreacted ethane and where applicable propane, and
- (ii) by compressing said rest of the gaseous mixture, e.g. by means of a gas compressor, so as to condense the unreacted ethane (and where applicable the propane), to separate and to isolate it (or them) in liquid form from methane which is preferably isolated in its turn in gaseous form.

It is also possible to perform a double (or multiple) fractionation by subjecting the above-mentioned gaseous mixture (M2) to a combination of two (or more) successive fractionations of a different type, chosen from the six above-mentioned types of fractionation.

A double fractionation of the above-mentioned gaseous mixture (M2) can, for example, be performed by combining two successive fractionations of a different type, namely:
- the one by change of physical state, preferably of liquid/gaseous phase of the mixture, in particular by distillation or by condensation, and the other by molecular filtration, preferably by means of semi-permeable and selective membrane, or
- the one by change of physical state, preferably of liquid/gaseous phase of the mixture, in particular by distillation or by condensation, and the other by adsorption, preferably by means of molecular sieve or any other adsorbent, or
- the one by change of physical state, preferably of liquid/gaseous phase of the mixture, in particular by distillation or by condensation, and the other by absorption, preferably by means of absorbing oil, or
- the one by change of physical state, preferably of liquid/gaseous phase of the mixture, in particular by distillation or by condensation, and the other by cryogenic expansion, preferably by means of expansion turbine, or
- the one by change of physical state, preferably of liquid/gaseous phase of the mixture, in particular by distillation or by condensation, and the other by compression, preferably by means of gas compressor, or
- the one by molecular filtration, preferably by means of semi-permeable and selective membrane, and the other, by adsorption, preferably by means of molecular sieve or any other adsorbent, or
- the one by molecular filtration, preferably by means of semi-permeable and selective membrane, and the other by absorption, preferably by means of absorbing oil, or
- the one by molecular filtration, preferably by means of semi-permeable and selective membrane, and the other by cryogenic expansion, preferably by means of expansion turbine, or
- the one by molecular filtration, preferably by means of semi-permeable and selective membrane, and the other by compression, preferably by means of gas compressor, or
- the one by adsorption, preferably by means of molecular sieve or any other adsorbent, and the other by absorption, preferably by means of absorbing oil, or
- the one by adsorption, preferably by means of molecular sieve or any other adsorbent, and the other by cryogenic expansion, preferably by means of expansion turbine, or
- the one by adsorption, preferably by means of molecular sieve or any other adsorbent, and the other by compression, preferably by means of gas compressor, or
- the one by absorption, preferably by means of absorbing oil, and the other by cryogenic expansion, preferably by means of expansion turbine, or
- the one by absorption, preferably by means of absorbing oil, and the other by compression, preferably by means of gas compressor, or
- the one by cryogenic expansion, preferably by means of expansion turbine, and the other by compression, preferably by means of gas compressor.

Each of said combinations can be performed in a given chronological order or in a reverse order.

Thus, for example, the two successive fractionations of a different type can be performed advantageously:
(i) by cooling the gaseous mixture (M2) to a temperature at which the higher alkanes (C₃₊), preferably (C₄₊) condense, so as to condense said higher alkanes, to separate and to isolate them in liquid form from the rest of the gaseous mixture comprising methane, unreacted ethane and where applicable propane, and
(ii) by passing said rest of the gaseous mixture through a semi-permeable and selective membrane, so as to arrest and to isolate the unreacted ethane and where applicable the propane, and to allow methane to pass through and preferably to be isolated in its turn in gaseous form.

It is also possible to perform the two above-mentioned successive fractionations, but in a reverse order, that is to say:
(i) by passing the gaseous mixture (M2) through a semi-permeable and selective membrane, so as to arrest and to isolate the higher alkanes (C₃₊), preferably(C₄₊), and to allow the rest of the gaseous mixture comprising methane, unreacted ethane and where applicable propane to pass through, and
(ii) by cooling said rest of the gaseous mixture to a temperature at which ethane and where applicable propane condense(s), so as to condense the unreacted ethane and where applicable the propane, and to separate and to isolate it (or them) in liquid form from methane which is preferably isolated in its turn in gaseous form.

Thus, e.g. by means of a double (or multiple) fractionation such as one of those described above, the methane can be isolated in stage (4) and preferably recycled into stage (1) for non-oxidative methane coupling. Furthermore, the unreacted ethane and where applicable the propane is/are preferably isolated in stage (4) and advantageously is/are recycled into stage (3) for ethane metathesis, in particular together or separately.

Stage (4) may also be performed as a simple compression step and as an integral part of stage (1) through which higher alkanes, such as the higher alkanes (C₃₊), preferably (C₄₊), are separated e.g. by condensation.

The process of the invention has the advantage of converting methane easily with a high yield into the higher alkanes (C₃₊), preferably (C₄₊), which are capable of being easily transportable in liquid form, in conditions relative close to standard temperature and pressure conditions. It also exhibits a very high "carbon atom efficiency" which can be equal to higher than 95%. Furthermore, it can advantageously produce great amounts of hydrogen which can be isolated and used for supplying sufficient thermal and/or electrical energies for running the process.

The following example illustrates the present invention.

### Example

### (1) Preparation of a metal catalyst (C1): an organometallic tantalum hydride grafted onto silica.

In a first stage, 50 mg of a silica sold under the trade name "Aerosil 200"® by Degussa (Germany) were subjected to a dehydroxylation treatment under an absolute pressure of 10⁻² Pa, at 700 °C, for 15 hours. At the end of this time, the silica thus treated was cooled to 25°C under an argon atmosphere.

In a second stage, 50 mg of the silica prepared beforehand were isolated and introduced into a reactor at 25 °C under an argon atmosphere. A precursor (Pr) was then introduced, namely tantalum tris(neopentyl)neopentylidene, of general formula (8):

Ta [- CH₂ - C(CH₃)₃]₃ [ = CH - C(CH₃)₃] (8)

The reactor was then heated to 70 °C for 2 hours, so as to sublime the precursor (Pr) onto the silica and to form an organometallic tantalum compound grafted onto the silica. At the end of this time, the surplus of unreacted precursor (Pr) was eliminated by inverse sublimation at 70°C. The reactor was then cooled to 25 °C under an argon atmosphere and the organometallic tantalum compound thus grafted onto the silica was isolated under argon. It contained 5.5 wt % of tantalum.

In a third stage, the organometallic tantalum compound grafted onto the silica prepared beforehand was subjected to a hydrogenolysis treatment by contacting with hydrogen, under an absolute hydrogen pressure of 73 kPa, at 150°C, for 15 hours. At the end of this time, a metal catalyst (C1) based on an organometallic tantalum hydride grafted onto silica and containing 5.5 wt % of tantalum was obtained and isolated under an argon atmosphere.

### (2) Preparation of a catalyst (C2): an organometallic tungsten hydride grafted onto alumina.

In a first stage, 2 g of an α-alumina containing 90 wt % of alumina and 9 wt % of water, and sold by Johnson Matthey (Great Britain), were subjected to a calcination treatment under a dry air current at 500°C for 15 hours, then to a dehydroxylation treatment under an absolute pressure of 10⁻² Pa, at 500 °C, for 15 hours. At the end of this time, the alumina thus treated was cooled under an argon atmosphere to 25 °C.

In a second stage, 2 g of the alumina prepared beforehand were isolated and introduced under an argon atmosphere into a reactor fitted with a magnetic agitator at 25°C. Then, 305 mg of a precursor (Pr'), namely tungsten tris(neopentyl)neo-pentylidyne of general formula (9) :

W [ - CH₂ - C(CH₃)₃]₃ [≡ C - C(CH₃)₃] (9)

were introduced.

The reactor was heated to 66 °C and the mixture thus obtained was agitated in the dry state for 4 hours. At the end of this time, the reactor was cooled to 25° C under an argon atmosphere, and the solid mixture was then washed with n-pentane at 25 °C. The solid compound thus washed was vacuum dried, then isolated under an argon atmosphere, so as to obtain an organometallic tungsten compound grafted onto alumina, containing 3.9 wt % of tungsten.

In a third stage, 40 mg of the organometallic tungsten compound grafted onto alumina prepared beforehand were isolated and subjected in a reactor to a hydro-genolysis treatment by contacting with hydrogen under an absolute hydrogen pressure of 73 kPa, at 150 °C, for 15 hours. At the end of this time, the reactor was cooled to 25°C, and there was obtained and isolated under an argon atmosphere a metal catalyst (C2) based on an organometallic tungsten hydride grafted onto alumina, containing 3.9 wt % of tungsten.

### (3) Conversion of methane into higher alkanes (C₄₊).

According to stage (1), methane was introduced continuously into a reactor (R1) heated to 475 °C, under an absolute total pressure of 5 MPa, and which contained the metal catalyst (C1) prepared beforehand. By non-oxidative methane coupling reaction, there formed in the reactor (R1) a gaseous mixture (M1) comprising ethane, hydrogen and unreacted methane. The gaseous mixture (M1) was withdrawn continuously out of the reactor (R1).

According to stage (2), the gaseous mixture (M1) withdrawn from the reactor (R1) was introduced continuously into a fractionation zone (F1) wherein ethane, hydrogen and unreacted methane were separated from each other continuously. The methane thus separated was isolated and recycled into the reactor (R1) where the methane coupling reaction was continued. During this time, the separated hydrogen was isolated in order to be used in part for activating and regenerating the catalysts (C1) and (C2), the other part being used as fuel for producing electrical energy.

According to stage (3), the ethane thus isolated was then introduced continuously into a reactor (R2) heated to 150 °C, under a total absolute pressure of 2 MPa, and containing the catalyst (C2) prepared beforehand. By ethane metathesis reactions, there formed in the reactor (R2) a gaseous mixture (M2) comprising higher alkanes (C₄₊), namely butanes, pentanes, hexanes, heptanes and octanes, and also methane, propane and unreacted ethane. The gaseous mixture (M2) was withdrawn continuously out of the reactor (R2).

According to stage (4), the gaseous mixture (M2) thus withdrawn from the reactor (R2) was then introduced continuously into a fractionation zone (F2) wherein the higher alkanes (C₄₊), the methane and the unreacted ethane in admixture with the propane were separated from each other. The unreacted ethane in admixture with the propane thus separated were isolated and recycled into the reactor (R2) where the ethane metathesis reactions were continued. During this time, the methane thus separated was also isolated and recycled into the reactor (R1) where the methane coupling reaction was continued.

## Claims

1. Process for converting methane into higher alkanes having at least 3 carbon atoms (C₃₊), preferably at least 4 carbon atoms (C₄₊), **characterised in that** it comprises the following stages:
- a stage (1) for non-oxidative methane coupling, comprising contacting methane with a metal catalyst (C1) capable of producing, in contact with alkane, reactions involving the splitting and recombining of carbon-carbon and/or carbon-hydrogen and/or carbon-metal bonds, so as to form a mixture (M1) comprising ethane and hydrogen,
- optionally a stage (2) comprising separating ethane from the mixture (M1), so as to isolate the ethane,
- a stage (3) for ethane metathesis, comprising contacting the ethane isolated in stage (2) or the mixture (M1) obtained in stage (1) with a metal catalyst (C2) capable of producing, in contact with alkane, reactions involving the splitting and recombining of carbon-carbon and/or carbon-hydrogen and/or carbon-metal bonds, said catalyst being identical to or different from the catalyst (C1), so as to form a mixture (M2) comprising methane and higher alkanes (C₃₊), preferably (C₄+), and
- a stage (4) comprising separating the higher alkanes (C₃₊), preferably (C₄+) from the mixture (M2), so as to isolate said higher alkanes.

2. Process according to claim 1, **characterised in that** the catalysts (C1) and (C2) are chosen from catalysts capable of alkane metathesis, preferably chosen from supported metal clusters, and especially from metal hydrides, organometallic compounds and organometallic hydrides, particularly supported on, more specifically grafted onto, a solid support.

3. Process according to claim 2, **characterized in that** the supported metal clusters comprise one or more metals of Groups 4, 5 and/or 6 of the Periodic Table of the Elements, preferably tantalum.

4. Process according to claim 2 or 3, **characterized in that** the supported metal clusters comprise a solid support and a metal cluster, preferably bonding between the metals of the metal cluster, and bonding between the metal cluster and the solid support.

5. Process according to claim 4, **characterized in that** the metal atom of the metal cluster is bonded to at least one other metal of the metal cluster, preferably to six or fewer metal atoms of the metal cluster.

6. Process according to any one of claims 2 to 5, **characterized in that** the supported metal clusters comprise a solid support chosen from a metal oxide, a refractory oxide, a molecular sieve, a zeolite, a metal phosphate and a material comprising a metal and oxygen, preferably silica.

7. Process according to claim 1 or 2, **characterised in that** the catalysts chosen from metal hydrides, organometallic compounds and organometallic hydrides comprise at least one metal chosen from lanthanides, actinides and metals of Groups 2 to 12, preferably transition metals of Groups 3 to 12 of the Periodic Table of the Elements.

8. Process according to claim 7, **characterised in that** the catalysts comprise at least one metal chosen from scandium, yttrium, lanthanum, titanium, zirconium, hafnium, vanadium, niobium, tantalum, chromium, molybdenum, tungsten, rhenium, ruthenium, osmium, nickel, iridium, palladium, platinum, cerium and neodymium, preferably from yttrium, titanium, zirconium, hafnium, vanadium, niobium, tantalum, chromium, molybdenum, tungsten, rhenium, ruthenium and platinum, more particularly from niobium, tantalum, molybdenum, tungsten and rhenium.

9. Process according to claim 7 or 8, **characterized in that** the catalysts comprise one or more ligands comprising at least one oxygen atom and/or at least one nitrogen atom, and preferably chosen from oxo, alkoxo, aryloxo, aralkyloxo, nitrido, imido and amido ligands.

10. Process according to any one of claims 7 to 9, **characterised in that** the catalysts comprise a solid support chosen from metal or refractory oxides, sulfides, carbides, nitrides and salts, and from carbon, mesoporous materials, organic/inorganic hybrid materials, metals and molecular sieves.

11. Process according to any one of claims 1 to 10, **characterised in that** stage (1) is performed at a temperature chosen in a range of from 150 to 700 °C, preferably 200 to 650 °C, in particular 300 to 600 °C.

12. Process according to any one of claims 1 to 11, **characterised in that** stage (1) is performed under an absolute total pressure chosen in a range of from 0.1 to 50 MPa, preferably 0.1 to 30 MPa, in particular 0.1 to 20 MPa.

13. Process according to any one of claims 1 to 12, **characterized in that** stage (1) is performed in a reaction zone comprising at least one reactor chosen from tubular or multiple-tubular reactors, distillation column reactors, slurry reactors, fluidised bed reactors, mechanically agitated bed reactors, fluidised and mechanically agitated bed reactors, fixed bed reactors, moving bed reactors and circulating bed reactors.

14. Process according to any one of claims 1 to 13, **characterised in that** stage (2) comprises one or more fractionations of the mixture (M1) chosen from: (i) fractionation by change of physical state, preferably of liquid/gaseous phase of the mixture, preferably by distillation or by condensation, in particular by means of distillation/condensation column or tower, (ii) fractionation by molecular filtration, preferably by means of semi-permeable and selective membrane, (iii) fractionation by adsorption, preferably by means of molecular sieve or any other adsorbent, (iv) fractionation by absorption, preferably by means of absorbing oil, (v) fractionation by cryogenic expansion, preferably by mean of expansion turbine, and (vi) fractionation by compression, preferably by means of gas compressor.

15. Process according to any one of claims 1 to 14, **characterized in that** stage (2) is performed in a fractionation zone which is either separate and distinct from a reaction zone (Z1) wherein stage (1) is performed, or located in a part of said reaction zone (Z1), preferably in a distillation column reactor used both for stage (1) and stage (2) and containing the catalyst (C1).

16. Process according to any one of claims 1 to 15, **characterized in that** hydrogen is separated and isolated from mixture (M1) in stage (2) and preferably used in one or more applications or stages, distinct or not from the present process, particularly used as an agent of activation or regeneration of the catalyst(s) (C1) and/or (C2), or as a fuel for producing thermal and/or electrical energies, in particular as a fuel in hydrogen fuel cells for producing electrical energy, such energies being preferably used for running the process, or as a fuel for automobile, or as a reagent in a chemical, petrochemical or refinery plant.

17. Process according to any one of claims 1 to 16, **characterized in that** the mixture (M1) in addition comprises unreacted methane which is separated and isolated in stage (2), and then recycled into stage (1).

18. Process according to any one of claims 1 to 17, **characterized in that** stages (1) and (2) are simultaneously performed in at least one distillation column reactor comprising both a reaction zone and a fractionation zone.

19. Process according to claim 18, **characterized in that** in the reaction zone, the contacting of the methane with the catalyst (C1) is performed so as to form the mixture (M1) preferably in a liquid/gaseous form, and simultaneously, in the fractionation zone, the separation of the ethane from the mixture (M1) is performed preferably by distillation/condensation, so as to isolate the ethane.

20. Process according to any one of claims 1 to 19, **characterised in that** stage (3) is performed at a temperature chosen in a range of from 20 to 400 °C, preferably 50 to 350 °C, in particular 70 to 300 °C.

21. Process according to any one of claims 1 to 20, **characterised in that** stage (3) is performed under an absolute total pressure chosen in a range of from 0.1 to 50 MPa, preferably 0.1 to 30 MPa, in particular 0.1 to 20 MPa.

22. Process according to any one of claims 1 to 21, **characterized in that** stage (3) is performed in the presence of hydrogen, preferably separated from the mixture (M1) in stage (2), in particular under a partial pressure of hydrogen chosen in a range of from 0.0001 to 10 MPa, preferably from 0.001 to 1 MPa.

23. Process according to any of claims 1 to 22, **characterized in that** stage (3) is performed in a reaction zone comprising at least one reactor chosen from tubular or multi-tubular reactors, distillation column reactors, slurry reactors, fluidised bed reactors, mechanically agitated bed reactors, fluidised and mechanically agitated bed reactors, fixed bed reactors, moving bed reactors and circulating bed reactors.

24. Process according to any one of claims 1 to 23, **characterised in that** stage (4) comprises one or more fractionations of the mixture (M2) chosen from: (i) fractionation by change of physical state, preferably of liquid/gaseous phase of the mixture, particularly by distillation or by condensation, in particular by means of distillation/condensation column or tower, (ii) fractionation by molecular filtration, preferably by means of semi-permeable and selective membrane, (iii) fractionation by adsorption, preferably by means of molecular sieve or any other adsorbent, (iv) fractionation by absorption, preferably by means of absorbing oil, (v) fractionation by cryogenic expansion, preferably by means of expansion turbine, and (vi) fractionation by compression, preferably by means of gas compressor.

25. Process according to any one of claims 1 to 24, **characterized in that** stage (4) is performed in a fractionation zone which is either distinct and separate from a reaction zone (Z2) wherein stage (3) is performed, or located in a part of said reaction zone (Z2), preferably in a distillation column reactor used both for stage (3) and stage (4) and containing the catalyst (C2).

26. Process according to any one of claims 1 to 25, **characterized in that** stages (3) and (4) are simultaneously performed in at least one distillation column reactor comprising a reaction zone and a fractionation zone.

27. Process according to claim 26, **characterized in that** in the reaction zone, the contacting of the ethane or of the mixture (M1) with the catalyst (C2) is performed so as to form the mixture (M2) preferably in a liquid/gaseous phase, and simultaneously, in the fractionation zone, the separation of the higher alkanes (C₃₊), preferably (C₄₊) from the mixture (M2) is performed preferably by distillation/condensation, so as to isolate said higher alkanes.

28. Process according to any one of claims 1 to 27, **characterized in that** the mixture (M2) in addition comprises unreacted ethane and where applicable propane, and **in that** the unreacted ethane and where applicable the propane is/are separated and isolated in stage (4) from the mixture (M2), and preferably is/are recycled into stage (3), in particular together or separately.

29. Process according to any one of claims 1 to 28, **characterized in that** methane is separated and isolated in stage (4), and then recycled into stage (1).
